# EUROPÄISCHE PATENTSCHRIFT

(11) **EP 3 866 706 B1**
(45) Veröffentlichungstag und Bekanntmachung des Hinweises auf die Patenterteilung: **02.07.2025**
(21) Anmeldenummer: 19791218.1
(22) Anmeldetag: 18.10.2019
(51) Int. Cl.: A61B 17/16, A61B 90/98, A61B 17/00

(54) **WERKZEUG MIT EINER IM SCHAFTBEREICH ANGEORDNETEN ARBEITSENDENBESTIMMUNGSVORRICHTUNG**
TOOL COMPRISING AN EFFECTOR END DETECTION DEVICE IN THE SHAFT REGION
OUTIL COMPRENANT UN DISPOSITIF DE DÉTECTION DE L'EXTRÉMITÉ DE TRAVAIL DANS LA RÉGION DE LA TIGE

(30) Priorität: 18.10.2018 DE 102018125884
(43) Veröffentlichungstag der Anmeldung: 25.08.2021
(73) Patentinhaber: Aesculap AG, 78532 Tuttlingen (DE)
(72) Erfinder: LENZENHUBER, Frederick, 78532 Tuttlingen (DE); BÜRK, Andre, 78056 Villingen-Schwenningen (DE)
(74) Vertreter: Winter, Brandl - Partnerschaft mbB
(86) Internationale Anmeldenummer: PCT/EP2019/078453
(87) Internationale Veröffentlichungsnummer: WO 2020/079268

(56) Entgegenhaltungen:
- EP-A1- 1 746 530
- EP-A1- 3 182 337
- DE-A1- 102011 052 501
- DE-A1- 102015 111 757
- DE-U1- 20 012 237
- US-A1- 2004 092 991
- US-A1- 2008 177 267
- US-A1- 2011 245 833
- US-A1- 2015 036 127

## Beschreibung

Die Erfindung betrifft ein Werkzeug oder Instrument, und bezieht sich insbesondere auf ein medizinisches/chirurgisches Werkzeug oder medizinisches/chirurgisches Instrument, das dazu konfiguriert ist, eine automatische und/oder automatisch dokumentierende Arbeitsendenbestimmung und/oder Arbeitsendenerkennung bereitzustellen.

### Stand der Technik

Bislang ist auf beispielsweise dem Gebiet der nichtmedizinischen elektrischen Handwerkzeugmaschinen eine Einrichtung zur automatischen Erkennung werkzeugspezifischer Daten von in die Handwerkzeugmaschine eingesetzten, auswechselbaren Werkzeugen bekannt, bei der ein Werkzeug wie etwa ein Schneidbohrer, ein Meißel oder dergleichen im Bereich eines Einsteckschafts eine geometrische Kodierung, beispielsweise eine mechanische Kodierung in Form von Kodierstufen, aufweist. Mittels einer in die Werkzeugaufnahme eingebauten und auf die Kodierung ausgerichteten Lesevorrichtung, etwa einem elektronischen, optischen oder mechanischen Sensor, werden der Kodierung entsprechende Signale erzeugt und zur Weiterverarbeitung an eine Steuereinheit einer Werkzeugmaschine geleitet.

Bei insbesondere im medizinischen Bereich vorkommenden Werkzeugen und/oder Instrumenten, wie beispielsweise Handwerkzeugen, sind im Wesentlichen drei Abschnitte unterscheidbar: Ein Griffabschnitt, ein Schaftabschnitt und zumindest ein Arbeitsende/Ettektor. Der Schaftabschnitt verbindet den Griffabschnitt mit dem Arbeitsende des Werkzeugs. Das Arbeitsende liegt dem Griffabschnitt am anderen Ende des Werkzeugs gegenüber, ist der eigentlich funktionelle Teil des Werkzeugs und kann in anwendungsspezifisch vielfältiger Weise ausgeformt sein. Werkzeuge der hierin in Bezug genommenen Art umfassen allgemein auf dem Gebiet der Medizin verwendbare Werkzeuge, Instrumente, Sprühdüsen, HF-Spitzen, Ultraschallklingen und dergleichen. Eine Beschränkung auf Produkte der vorgenannten Art aus speziell dem medizinischen Bereich besteht insoweit allerdings nicht.

Auf dem Gebiet der Medizin ist aufgrund der Art der eingesetzten Werkzeuge und/oder Instrumente sowie Bedingungen jeweiliger Arbeitsumgebungen eine wie vorstehend erwähnt koppelnde Kodierungserfassung weder darstellbar noch zweckmäßig, sondern es wird bislang mit Labeln bzw. Aufklebern gearbeitet, die am Werkzeug selbst oder auch auf einer Umverpackung des Werkzeugs angeordnet sein können. Um beispielsweise zu erkennen, welches Werkzeug gesteckt ist, muss das Label des bzw. jedes einzelnen Werkzeugs oder dessen Umverpackung gesichtet werden. Bisher existiert mithin keine automatische und/oder automatisch dokumentierende Arbeitsendenbestimmung oder Arbeitsendenerkennung, so dass ein Benutzer oder Kunde anhand von Labels bzw. beschriftenden Etiketten erkennen muss oder nur daran erkennen kann, um welches Produkt/Arbeitsende es sich handelt, und sodann manuell dokumentieren muss.

Davon nachteilig betroffen sind sowohl Benutzer als auch Händler bzw. Kunden und Hersteller solcher Werkzeuge. Unter anderem kann ein Benutzer oder Kunde eines solchen Werkzeugs beispielsweise ohne Sichten des Labels bzw. der Umverpackung nicht ohne weiteres erkennen, ob ein beabsichtigtes Werkzeug für seine spezifische Anwendung geeignet ist oder nicht. Ein Händler oder Kunde kann beispielsweise in der Regel ohne Inventur nicht erfassen, welche Produktbestände sich noch an einem Lager oder Konsignationslager befinden. Ein Hersteller kann beispielsweise nicht ermitteln, welche Produkte kombiniert und/oder verwendet wurden, kann eine Überlastung von Werkzeugen und gegebenenfalls damit verbundene Produktschäden nicht nachvollziehen, und kann für einen Kunden keine maßgeschneiderte Logistik bereitstellen.

Es besteht daher (nicht nur, aber insbesondere auf dem Gebiet der Medizin bzw. Medizintechnik) Bedarf an einer automatischen Erkennung von Werkzeugen und/oder Instrumenten, Sprühdüsen, HF-Spitzen, Ultraschallklingen und dergleichen, mittels welcher die vorgenannten Nachteile beseitigt und damit verbundene Probleme gelöst werden.

Relevanter Stand der Technik ist zum Beispiel in den Druckschriften DE 10 2015 111757 A1, US 2011/0245833 A1, US 2004/0092991 A1 und EP 3 182 337 A1 offenbart.

### Kurzbeschreibung der Erfindung

Der Erfindung liegt daher als eine Aufgabe zugrunde, ein Werkzeug wie beispielsweise ein medizinisches Werkzeug bereitzustellen, dessen proximales Arbeitsende bei beispielsweise einem Steckvorgang (in ein Handstück, Griff) erkannt werden kann. Darüber hinaus soll es möglich sein, Daten, beispielsweise Daten mit Bezug zu dem Arbeitsende und/oder darüber hinaus gehend weitere Daten einem Benutzer oder Kunden anzuzeigen bzw. an diesen zu übermitteln.

Erfindungsgemäß wird diese Aufgabe durch ein Werkzeug mit den Merkmalen des Anspruchs 1 gelöst. Vorteilhafte Weiterbildungen der Erfindung sind Gegenstand der beigefügten Unteransprüche.

### Kurzbeschreibung der Offenbarung

Der Erfindung liegt die allgemeine Idee zugrunde, an einem Werkzeug, insbesondere medizinischen (Bohr-, Fräs-, Schraub-) Werkzeug eine (Erkennungs-, Identifikations-, Kommunikations-) Vorrichtung anzuordnen, die eine Bestimmung oder Erkennung eines jeweils in ein Handstück/Griff/Antriebseinheit gesteckten Arbeitsendes (bzw. Werkzeugs) sowie eine Übermittlung von weiteren, wahlfrei zuvor auf einen Datenträger und/oder in eine Speichereinrichtung geschriebenen Daten, d.h. eine Arbeitsendenerkennung in Verbindung mit einem auslesbaren Datenspeicher (unter Verwendung einer Kommunikationsvorrichtung mit geringem Stromverbrauch auf Grundlage beispielsweise von Bluetooth, RFID, NFC und dergleichen) zusammen mit dem Arbeitsende, ermöglicht.

Die Erfindung sieht dazu vor, an dem Werkzeug eine dessen Arbeitsende bestimmende Vorrichtung mit zumindest einer Datenspeichervorrichtung bzw. einem Datenspeicherbereich einer vorbestimmten Datenspeicherkapazität anzuordnen, wobei in dem Datenspeicher gespeicherte Daten derart Bezug zu dem Arbeitsende haben, dass sie das Arbeitsende bestimmen und/oder zu einer Erkennung desselben heranziehbar sind, und darüber hinaus der Datenspeicher weitere Daten, beispielsweise anwendungsbezogene oder prozessbezogene Daten und dergleichen, vorhalten kann. Die Vorrichtung ist vorzugsweise eine Kommunikationsvorrichtung mit geringem Stromverbrauch, mit welcher Geräte in einem Kommunikationsbereich von typisch einigen Metern bis hin zu einigen 10 Metern vernetzbar sind oder drahtlos Daten übertragen und/oder austauschen können. Weiter vorzugsweise kann die Kommunikationsvorrichtung eine Bluetooth-Vorrichtung oder RFID-Vorrichtung sein. Alternativ sieht die Erfindung vor, einen Widerstand und/oder eine Speichervorrichtung in das Werkzeug integriert bereitzustellen, wobei der Widerstand für eine Bestimmung des Arbeitsendes ausgelegt oder spezifiziert ist, und die Speichervorrichtung weitere Daten, beispielsweise anwendungsbezogene oder prozessbezogene Daten und dergleichen, vorhalten kann.

Anwendungsbezogene, produktbezogene oder prozessbezogene Daten können beispielsweise eine Artikelnummer und/oder eine graphische Darstellung des Artikels, eine LOT-Angabe, d.h. Information über einen zusammengestellten Posten einer bestimmten Ware, ein MHD bzw. Mindesthaltbarkeitsdatum und dergleichen sein.

Mit derart anwendungsbezogenen oder prozessbezogenen Daten wird ein Benutzer oder Kunde in Verbindung mit einer Datenverarbeitungsvorrichtung in die Lage versetzt, in mannigfaltiger Weise Nutzungen zu dokumentieren. Beispielsweise kann dem Kunden auf einer Anzeigevorrichtung, beispielsweise einer Anzeige eines externen Geräts oder eines portablen Computers, Tablets, Smartphones oder anderweitigen Endgeräts angezeigt werden, um welches Produkt es sich handelt. Ferner kann dem Benutzer oder Kunden lagerbestandsbezogene Information angezeigt werden und/oder bei einem Lagerbestand unterhalb einer vorbestimmbaren Anzahl eine automatische Nachlieferung ausgelöst werden (Meldung beispielsweise "Ihr Konsignationslager ist bald erschöpft. Berechnet auf den Verbrauch der letzten vier Wochen wird Ihnen ein Bedarf von 50 Stück angeliefert. Tracking ID: 123456, Lieferung mittels Zustelldienst"), kann der Benutzer oder Kunde über einen unerwünschten Zustand informiert und zugunsten einer Verbesserung angeleitet werden (Meldung beispielsweise "Vorsicht: Überlastung des Handstücks festgestellt. Zu hoher Anpressdruck. Bitte verwenden Sie nach Möglichkeit ein anderes Arbeitsende"), oder können dem Benutzer oder Kunden Diagnoseinformationen bereitgestellt werden (Meldung beispielsweise "Überlastung des Handstücks detektiert. Die verwendeten Arbeitsenden im Erfassungszeitraum waren Typ A, Typ B, ... Dabei wurden X zu hohe Stromaufnahmen festgestellt" (X kann hierbei eine numerische Zahl sein)). Darüber hinaus ist auch ein direktes Protokollieren verwendeter Komponenten in analoger und/oder digitaler Form, etwa in einer IT-Infrastruktur mit beispielsweise Speicherplatz, Rechenleistung und/oder Anwendungssoftware lokal und/oder über das Internet (Cloud), in dezentralisierten Datenbanken, in Serverstrukturen oder dergleichen, und/oder in einer Patientenakte darstellbar.

Erfindungsgemäß resultieren somit vorteilhaft eine direkte, automatisierte Werkzeugerkennbarkeit für einen Benutzer, Kunden und/oder einen Hersteller, eine Übermittelbarkeit beliebige vieler weiterer Daten an einen Benutzer, Kunden und/oder einen Hersteller, und die Möglichkeit eines unmittelbaren Schreibens in eine beispielsweise elektronische Patientenakte für einen Benutzer oder Kunden.

Im Einzelnen wird die Aufgabe gelöst durch ein Werkzeug mit einer in einem Schaftbereich desselben angeordneten Arbeitsendenbestimmungsvorrichtung zur Bestimmung eines Arbeitsendes des Werkzeugs, wobei die Arbeitsendenbestimmungsvorrichtung eine beschreibbare und/oder auslesbare Datenspeichervorrichtung beinhaltet, die dazu konfiguriert ist, in zumindest einem ersten Speicherbereich zumindest Daten mit Bezug zu dem Arbeitsende des Werkzeugs zu speichern, und wobei die Arbeitsendenbestimmungsvorrichtung dazu angeordnet ist, die Daten mit Bezug zu dem Arbeitsende des Werkzeugs zur Erkennung des Arbeitsendes des Werkzeugs extern erfassbar bereitzustellen.

Bevorzugt ist die Arbeitsendenbestimmungsvorrichtung eine Kommunikationsvorrichtung mit geringem Stromverbrauch, die zumindest die Datenspeichervorrichtung und eine Antennenvorrichtung umfasst.

Auch bevorzugt ist die Kommunikationsvorrichtung mit geringem Stromverbrauch in einem Hohlkörper aufgenommen, der an einem dem Arbeitsende gegenüberliegenden Griff- oder Schaftende des Werkzeugs das Griff- oder Schaftende verlängernd angeordnet ist.

Weiter bevorzugt ist die Kommunikationsvorrichtung mit geringem Stromverbrauch an einem dem Arbeitsende gegenüberliegenden Griff- oder Schaftende des Werkzeugs in dieses eingelassen angeordnet.

Alternativ bevorzugt ist die Kommunikationsvorrichtung mit geringem Stromverbrauch in einem einen Schaftabschnitt oder einen Griffabschnitt umgreifenden Ringkörper aufgenommen.

Vorteilhaft kann dabei die Antennenvorrichtung in dem die Kommunikationsvorrichtung mit geringem Stromverbrauch aufnehmenden Ringkörper spulenförmig ausgebildet sein, und/oder kann die Kommunikationsvorrichtung mit geringem Stromverbrauch aufnehmende Ringkörper als eine umspritzte und/oder vergossene Baugruppe ausgebildet sein.

Alternativ bevorzugt ist die Kommunikationsvorrichtung mit geringem Stromverbrauch ringförmig auf einem Außenring einer an dem Werkzeug angeordneten Lagervorrichtung angeordnet, wobei auf einem ersten Teilabschnitt des Außenrings der Lagervorrichtung die beschreibbare und/oder auslesbare Datenspeichervorrichtung und auf einem zweiten Teilabschnitt des Außenrings der Lagervorrichtung die Antennenvorrichtung angeordnet ist, und wobei die Antennenvorrichtung spulenförmig ausgebildet ist. Besonders vorteilhaft ist in diesem Fall, dass der Außenring der Lagervorrichtung ortsfest gehalten ist und daher nicht mit rotiert, so dass keine Vibrationen aufgrund einer etwaigen Unwucht und dergleichen zu erwarten sind.

Alternativ bevorzugt ist die Kommunikationsvorrichtung mit geringem Stromverbrauch in einem ein Trägerelement bildenden hülsen- oder ringförmigen Distanzstück zur Positionierung auf einem Außenumfang des Werkzeugs aufgenommen, wobei in einem ersten Teilabschnitt des Distanzstücks die beschreibbare und/oder auslesbare Datenspeichervorrichtung und in einem zweiten Teilabschnitt des Distanzstücks die Antennenvorrichtung angeordnet ist, und wobei die Antennenvorrichtung spulenförmig ausgebildet ist.

Alternativ bevorzugt ist die Kommunikationsvorrichtung mit geringem Stromverbrauch in einem einen Spritzschutz bildenden Element auf einem Außenumfang des Werkzeugs aufgenommen, wobei in einem ersten Teilabschnitt des Spritzschutzelements die beschreibbare und/oder auslesbare Datenspeichervorrichtung und in einem zweiten Teilabschnitt des Spritzschutzelements die Antennenvorrichtung angeordnet ist, und wobei die Antennenvorrichtung spulenförmig ausgebildet ist.

Dabei kann vorteilhaft das den Spritzschutz bildende Element ein Spritzschutzkörper für eine an dem Werkzeug angeordnete Lagervorrichtung sein.

Darüber hinaus vorteilhaft und bevorzugt kann die Kommunikationsvorrichtung mit geringem Stromverbrauch eine RFID-Vorrichtung oder eine Bluetooth Low Energy-Vorrichtung sein.

Erfindungsgemäss ist die Arbeitsendenbestimmungsvorrichtung eine in das Werkzeug integrierte Einheit, die über eine elektrische Kontaktierung in einem Handstückbereich des Werkzeugs mit einem externen Steuergerät verbindbar ist.

Dabei kann vorteilhaft die in das Werkzeug integrierte Einheit einen das Arbeitsende des Werkzeugs bestimmenden Widerstand und/oder eine Daten mit Bezug zu dem Arbeitsende speichernde Speichervorrichtung umfassen.

Bevorzugt erstreckt sich die in das Werkzeug integrierte Einheit ausgehend von einem griff- oder schaftseitigen Ende des Werkzeugs mit kanalförmiger Struktur in Richtung hin zu dem Arbeitsende in das Werkzeug, wobei in der kanalförmigen Struktur an einem ersten, dem Arbeitsende zugewandten Ende der das Arbeitsende des Werkzeugs bestimmende Widerstand und/oder die Daten mit Bezug zu dem Arbeitsende speichernde Speichervorrichtung angeordnet ist und an einem zweiten, dem Arbeitsende abgewandten Ende die elektrische Kontaktierung angeordnet ist, wobei zumindest ein Leiterabschnitt den Widerstand und/oder die Speichervorrichtung elektrisch kontaktiert und wobei die kanalförmige Struktur von einem Isolator umhüllt und durch diesen gegenüber dem Werkzeug elektrisch isoliert ist.

Dabei kann vorteilhaft die beschreibbare und/oder auslesbare Datenspeichervorrichtung zumindest einen zweiten Speicherbereich beinhalten, der dazu konfiguriert ist, vorbestimmte andere Daten als die in dem zumindest einen ersten Speicherbereich gespeicherten Daten, die die Daten mit Bezug zu dem Arbeitsende des Werkzeugs sind, zu speichern.

### Kurzbeschreibung der Figuren

Die Erfindung wird nachstehend unter Bezugnahme auf die beigefügte Zeichnung näher beschrieben. Es zeigen:
Fig. 1 eine schematische Darstellung eines Werkzeugs mit einer Arbeitsendenbestimmungsvorrichtung gemäß einem ersten Beispiel (nicht durch die beanspruchte Erfindung abgedeckt);
Fig. 2 eine schematische Darstellung eines Werkzeugs mit einer Arbeitsendenbestimmungsvorrichtung gemäß einem zweiten Beispiel (nicht durch die beanspruchte Erfindung abgedeckt);
Fig. 3, 3a, 3b eine schematische Darstellung eines Werkzeugs mit einer Arbeitsendenbestimmungsvorrichtung gemäß einem dritten Beispiel (nicht durch die beanspruchte Erfindung abgedeckt);
Fig. 4 eine schematische Darstellung eines Werkzeugs mit einer Arbeitsendenbestimmungsvorrichtung gemäß einem vierten Beispiel (nicht durch die beanspruchte Erfindung abgedeckt);
Fig. 5 eine schematische Darstellung eines Werkzeugs mit einer Arbeitsendenbestimmungsvorrichtung gemäß einem fünften Beispiel (nicht durch die beanspruchte Erfindung abgedeckt);
Fig. 6 eine schematische Darstellung eines Werkzeugs mit einer Arbeitsendenbestimmungsvorrichtung gemäß einem sechsten Beispiel (nicht durch die beanspruchte Erfindung abgedeckt);
Fig. 7 eine schematische Darstellung eines Werkzeugs mit einer Arbeitsendenbestimmungsvorrichtung in einer Ausführungsform gemäß einem Ausführungsbeispiel der vorliegenden Erfindung; und
Fig. 8, 8a-d eine auszugsweise Schnittansicht zur Darstellung einer in dem Ausführungsbeispiel in das Werkzeug als integrierte Einheit ausgebildeten Arbeitsendenbestimmungsvorrichtung.

In den Figuren bezeichnen gleiche Bezugszeichen gleiche oder zumindest äquivalente Teile und Komponenten. Zweckmäßig wird insoweit eine mehrfach redundante Beschreibung solcher Teile und Komponenten weggelassen.

### Figurenbeschreibung

Eine Arbeitsendenbestimmungsvorrichtung bedeutet hierin eine an einem Werkzeug, beispielsweise einem Werkzeug zu medizinischen Zwecken wie z.B. ein in ein Antriebshandstück drehbar einsteckbares Bohr- Fräs- oder Schraubwerkzeug, angeordnete Vorrichtung zur Bestimmung bzw. Erkennung zumindest eines Arbeitsendes des (medizinischen) Werkzeugs. Eine Arbeitsendenerkennungsvorrichtung ist daher insoweit synonym bzw. gleichbedeutend zu der Arbeitsendenbestimmungsvorrichtung. Dazu hält, speichert oder beinhaltet die Arbeitsendenbestimmungsvorrichtung Daten, die das Arbeitsende des Werkzeugs bestimmen, d.h. Daten mit Bezug zu dem Arbeitsende des Werkzeugs, und ist dazu konfiguriert, diese Daten extern erfassbar nach außerhalb des Werkzeugs auszugeben oder zu signalisieren, in einer Weise derart, dass eine externe Vorrichtung, die mit der Arbeitsendenbestimmungsvorrichtung kommunikationsfähig in Verbindung steht, anhand der ausgegebenen Daten oder Signalisierung das sich an dem Werkzeug befindende Arbeitsende erfassen kann. Darüber hinaus ist die Arbeitsendenbestimmungsvorrichtung dazu konfiguriert, zusätzlich zu den Daten mit Bezug zu dem Arbeitsende weitere Daten, beispielsweise anwendungsbezogene, produktbezogene oder prozessbezogene Daten zu speichern und bedarfsweise ebenfalls an die externe Vorrichtung auszugeben.

Anwendungsbezogene, produktbezogene oder prozessbezogene Daten wie vorstehend erwähnt können beispielsweise eine Artikelnummer und/oder eine graphische Darstellung des Artikels, eine LOT-Angabe, d.h. Information über einen zusammengestellten Posten einer bestimmten Ware, ein MHD bzw. Mindesthaltbarkeitsdatum und dergleichen sein.

Mit derart anwendungsbezogenen oder prozessbezogenen Daten wird ein Benutzer oder Kunde in Verbindung mit einer Datenverarbeitungsvorrichtung in die Lage versetzt, in mannigfaltiger Weise Nutzungen zu dokumentieren. Beispielsweise kann dem Kunden auf einer Anzeigevorrichtung, beispielsweise einer Anzeige eines externen Geräts eines portablen Computers, Tablets, Smartphones, Virtual Reality, Augmented Reality oder anderweitigen Endgeräts angezeigt werden, um welches Produkt es sich handelt. Ferner kann dem Benutzer oder Kunden lagerbestandsbezogene Information angezeigt werden und/oder bei einem Lagerbestand unterhalb einer vorbestimmbaren Anzahl eine automatische Nachbestellung ausgelöst werden (Meldung beispielsweise "Ihr Konsignationslager ist bald erschöpft. Berechnet auf den Verbrauch der letzten vier Wochen wird Ihnen ein Bedarf von 50 Stück angeliefert. Tracking ID: 123456, Lieferung mittels Zustelldienst"), kann der Benutzer oder Kunde über einen unerwünschten Zustand informiert und zugunsten einer Verbesserung angeleitet werden (Meldung beispielsweise "Vorsicht: Überlastung des Handstücks festgestellt. Zu hoher Anpressdruck. Bitte verwenden Sie nach Möglichkeit ein anderes Arbeitsende"), oder können dem Benutzer oder Kunden Diagnoseinformationen bereitgestellt werden (Meldung beispielsweise "Überlastung des Handstücks detektiert. Die verwendeten Arbeitsenden im Erfassungszeitraum waren Typ A, Typ B, ... Dabei wurden X zu hohe Stromaufnahmen festgestellt"). Darüber hinaus ist auch ein direktes Protokollieren verwendeter Komponenten in etwa einer Patientenakte darstellbar.

Die Übertragung, d.h. die Ausgabe jeweiliger der vorgenannten Daten erfolgt in dem vorliegenden ersten Beispiel unter Verwendung einer Kommunikation (bzw. Kommunikationsvorrichtung oder Kommunikationsverfahren), auf Grundlage beispielsweise einer Übertragungstechnik oder Funktechnik mit geringem Stromverbrauch, mit welcher Geräte in einer Nahfeldumgebung, typischerweise in einem Kommunikationsbereich von einigen Meter bis hin zu einigen 10 Metern, vernetzbar sind oder drahtlos Daten übertragen und/oder austauschen können. Beispiele für eine solche Übertragungstechnik sind Bluetooth Low Energy, Bluetooth LE (kurz BLE) oder Bluetooth Smart als eine den Industriestandard Bluetooth ergänzende Erweiterung, RFID oder NFC. In diesem Beispiel sind dabei Kommunikationskomponenten der Übertragungstechnik mit geringem Stromverbrauch, wie beispielsweise eine Bluetooth (Low Energy)-Vorrichtung (BLE) oder eine RFID-Vorrichtung, beispielsweise ein RFID-Chip bzw. Transponder oder Tag mit vorbestimmter Datenspeicherkapazität, in der Arbeitsendenbestimmungsvorrichtung aufgenommen und angeordnet. Eine jeweilige Vorrichtung umfasst darüber hinaus zumindest eine Antenne zur drahtlosen Übertragung der Daten. Bluetooth an sich ist gut dokumentiert, so dass weitergehende Einzelheiten hierin nicht redundant beschrieben werden.

Unter einer RFID-Vorrichtung ist hierin eine Funkfrequenzidentifikationsvorrichtung (RFID = Radio Frequency Identification) zu verstehen. Auf einem RFID-Chip können allgemein die unterschiedlichsten Informationen gespeichert werden, und ein Lesegerät kann diese Daten bzw. Informationen jederzeit über Funk auslesen. Bei Nahfeldkommunikation bzw. NFC (Near Field Communication) in Geräten wie beispielsweise Smartphones und dergleichen handelt es sich um eine spezielle Version von RFID.

RFID-Chips bzw. Transponder beinhalten in der Regel einen Mikrochip, eine Antenne, die vorzugsweise in Form einer Spule ausgeführt ist, mit einem Durchmesser abhängig von einer zu erzielenden Reichweite, einem Träger oder Gehäuse zum Schutz der Transponderelektronik vor der Umgebung, und bei aktiven RFID-Chips eine Energiequelle, beispielsweise eine Batterie. Bei passiven RFID-Chips erfolgt die Energieversorgung über die Antenne von außen, d.h. RFID/NFC kann passiv arbeiten und benötigt keine Batterie oder anderweitige Stromversorgung, sondern kann aus einer von einem Scanner auf einer Radiofrequenz ausgesendeten Welle mit Energie versorgt werden, diese in einem kleinen Kondensator speichern und so einen Mikrocontroller auf dem Chip mit Strom versorgen. Die Übermittlung von Daten an den Scanner kann unter Verwendung der sogenannten Lastmodulation, bei der ein Feld des Scanners stärker oder schwächer belastet wird, wobei diese Lastschwankungen von dem Scanner als serielle Daten registrierbar sind, oder unter Verwendung des Prinzips der "modulierten Rückstreuung", bei welcher Nebenfrequenzen von dem Scanner als digitale Daten interpretierbar zurückgestreut werden, erfolgen.

Eine zur Verfügung stehende Speicherkapazität des beschreibbaren Speichers eines RFID-Chips kann von wenigen Bit bis zu mehreren KBytes reichen. Der Datensatz des Transponders wird bei dessen Herstellung fest in ihm als laufende eindeutige Zahl (inhärente Identität) oder bei dessen Applikation als nicht einmalige Daten abgelegt. Inzwischen verfügbare Tags können auch später geändert oder mit weiteren Daten beschrieben werden. Beschreibbare Transponder können dazu als Speichertechnologien nicht-flüchtige Speicher, bei welchen Daten ohne Stromversorgung erhalten bleiben und die daher vorzugsweise für induktiv versorgte RFID geeignet sind, wie beispielsweise EEPROM oder FRAM, und/oder flüchtige Speicher, welche eine ununterbrochene Stromversorgung benötigen, um die Daten zu behalten, wie beispielsweise SRAM verwenden.

RFID ist im Stand der Technik gut dokumentiert und wird daher hierin nicht weiter beschrieben. Es versteht sich jedoch, dass RFID wie hierin verwendet nicht auf die vorstehend beispielhaften Ausführungen und Verkörperungen beschränkt ist.

Die folgende Beschreibung von Beispielen bezieht sich auf jeweils eine RFID-Vorrichtung als Kommunikationsvorrichtung mit geringen Stromverbrauch verwendende Ausführungsformen. Eine Beschränkung hierauf besteht jedoch nicht. Es versteht sich, dass anstelle der RFID-Vorrichtung eine Bluetooth-Vorrichtung, beispielsweise eine Bluetooth Low Energy-Vorrichtung (BLE), oder eine andere Kommunikationsvorrichtung mit vorbestimmbar geringem Stromverbrauch verwendet werden kann.

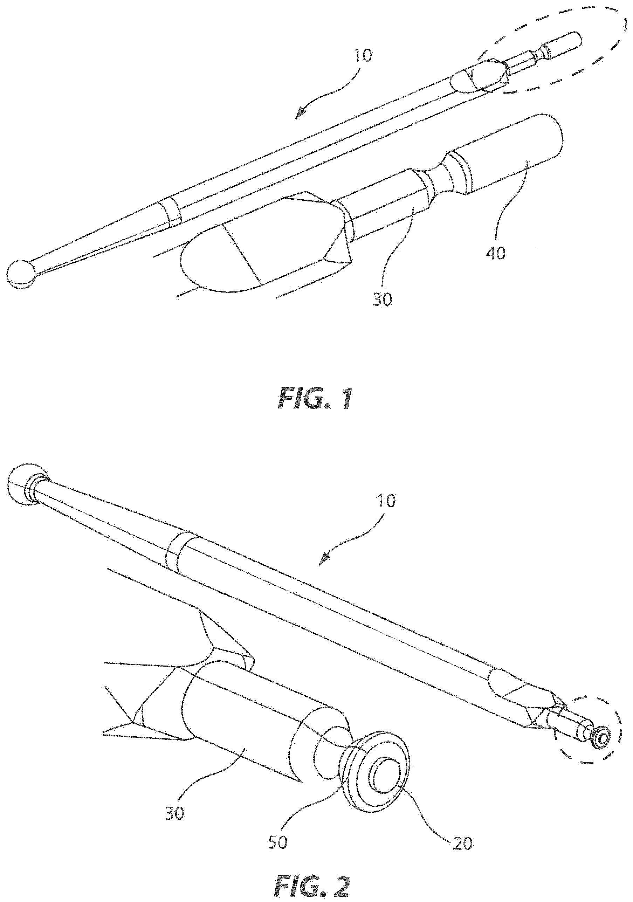

Fig. 1 stellt als ein erstes Beispiel ein Werkzeug mit auslesbarem Datenspeicher dar, bei dem eine RFID-Vorrichtung (nicht dargestellt) an einem Werkzeug 10, vorzugsweise ein Fräswerkzeug mit distalem Fräskopf, einem Werkzeugschaft und einem proximalen Einsteck-Endabschnitt an einem von dem Arbeitsende (Fräskopf) entfernten Ende (Einsteck-Endabschnitt) des Schaftabschnitts bzw. Griff(-einsteck-) abschnitts 30, d.h. schaftendseitig bzw. griffseitig, angeordnet ist.

Der Einsteck-Endabschnitt weist vorliegend ein unrundes Profil für eine Drehmomenteinleitung/-übertragung in den Werkzeugschaft auf. Proximal zu dem Abschnitt mit unrundem Profil ist die RFID-Vorrichtung angeordnet, derart, dass sich diese außerhalb des Drehmoment-Übertragungszugs befindet und somit Drehmomentunbelastet bleibt.

Die RFID-Vorrichtung mit Antenne (nicht dargestellt) und Transponderelektronik (nicht dargestellt) ist in dem ersten Beispiel in beispielsweise einem einseitig offenen Gehäuse 40, das rundrohrförmig ("ampullenförmig") ausgeführt sein kann, verbaut und aufgenommen, und das Gehäuse 40 ist vorzugsweise fluid- und gasdicht in geeigneter Weise an dem proximalen Ende des Werkzeugs 10 angelenkt/fixiert/stoffeinstückig ausgeformt.

Fig. 2 stellt als ein zweites Beispiel ebenfalls ein Werkzeug mit auslesbarem Datenspeicher dar, bei dem die RFID-Vorrichtung 20 in eine eine Art GehäuseInnenraum ausbildende Ausnehmung an dem Werkzeug 10 an einem von dem Arbeitsende entfernten proximalen Ende des Schaftabschnitts bzw. Griffabschnitts 30, d.h. schaftendeseitig bzw. griffseitig, eingelassen, d.h. in das Werkzeug versenkt, angeordnet ist. Die RFID-Vorrichtung 20 mit Antenne (nicht dargestellt) und Transponderelektronik (nicht dargestellt) ist in dem zweiten Beispiel in beispielsweise der Ausnehmung, die einseitig offen und mit einem die Öffnung fluid- und gasdicht verschließenden Abdeckelement ausgeführt sein kann, aufgenommen, und die Ausnehmung ist vorzugsweise in geeigneter Weise und Form, beispielsweise als ein griffgünstig rundförmiger Fortsatz 50 mit in etwa dem Durchmesser des Werkzeugs am werkzeugendseitigen Anlenkungsort angelenkt/fixiert.

An dieser Stelle sei darauf hingewiesen, dass der Fortsatz 50 gemäß der Fig. 2 nur schematisch dargestellt ist und natürlich zumindest hinsichtlich seiner axialen Abmessung an die in dessen Ausnehmung aufgenommene RFID-Vorrichtung angepasst sein muss.

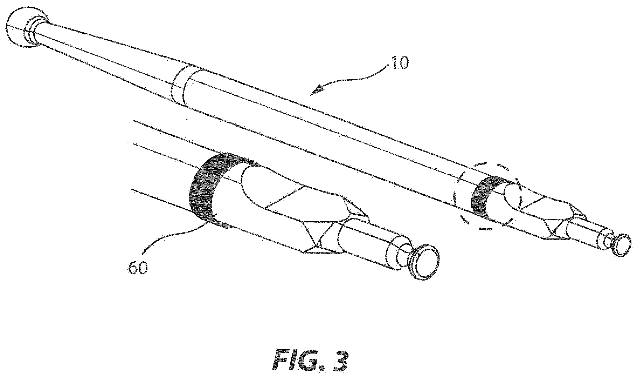

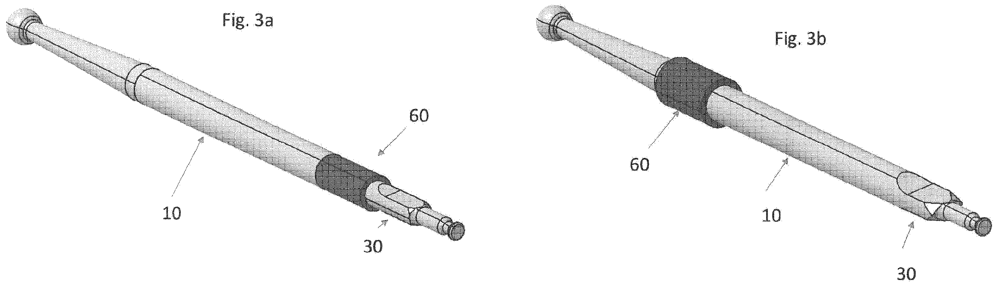

Fig. 3 stellt als ein drittes Beispiel ebenfalls ein Werkzeug mit auslesbarem Datenspeicher dar, bei dem die RFID-Vorrichtung in einem am von dem distalen Arbeitsende entfernten Endabschnitt des Schaftabschnitts/Werkzeugschafts bzw. Griffabschnitts 30, d.h. schaftendseitig bzw. griffseitig an dem Werkzeug 10 ortsfest, beispielsweise rastend, angebrachten oder über dieses schiebbar beweglichen Ringkörper 60 aufgenommen angeordnet ist. Die RFID-Vorrichtung mit Antenne (nicht dargestellt) und Transponderelektronik (nicht dargestellt) ist in dem dritten Beispiel in beispielsweise dem Volumen des Ringkörpers 60 aufgenommen. Zur fluid- und gasdichten Ausführung des Ringkörpers 60 kann dieser vorzugsweise als umspritzte und/oder vergossene Baugruppe aus beispielsweise einem Kunststoffmaterial oder dergleichen erzeugt sein. Außerdem kann die Antenne der RFID-Vorrichtung im Ringraum des Ringkörpers 60 spulenförmig ausgebildet sein.

In Ergänzung zu der vorstehend beschriebenen Fig. 3 sei zusätzlich noch auf die Fig. 3a und 3b verwiesen. In diesen weiteren Figuren ist das medizinische Werkzeug 10 gemäß der Fig. 3 gezeigt, bei welchem der aus der Fig. 3 bereits bekannte Ringkörper 60 zur Aufnahme der RFID-Vorrichtung an unterschiedlichen Axialpositionen des Werkzeugschafts gelagert ist. So ist es beispielsweise möglich, den Ringkörper 60 unmittelbar distal zu dem unrunden proximalen Einsteck-Endabschnitt des Werkzeugs 10 oder in einem Mittenabschnitt des Werkzeugschafts zu lagern. Auch zeigen die Fig. 3a und 3b, dass der Ringkörper 60 gemäß der Fig. 3 zumindest hinsichtlich seiner Axialabmessungen variieren kann, um möglichst optimal an die Abmessungen der darin aufgenommenen RFID-Vorrichtung angepasst zu sein.

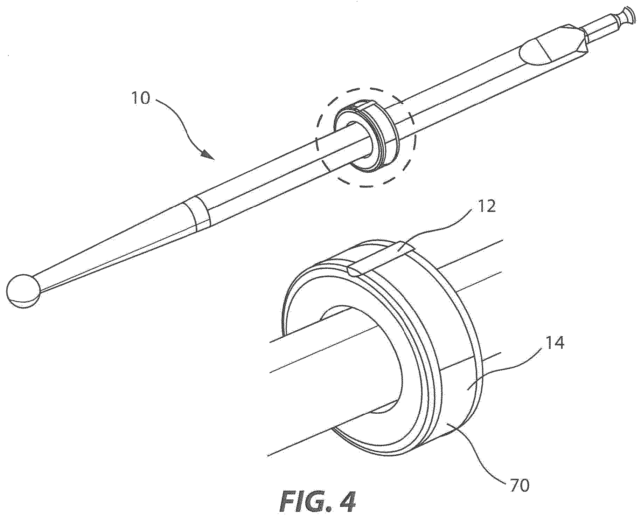

Fig. 4 stellt als ein viertes Beispiel ebenfalls ein Werkzeug mit auslesbarem Datenspeicher dar, bei dem die RFID-Vorrichtung auf einem Außenring einer entlang des Schaftabschnitts vorzugsweise fest verbaut angeordneten Lagervorrichtung 70, beispielsweise einem Kugellager, aufgenommen angeordnet ist. Die RFID-Vorrichtung mit Antenne und Transponderelektronik (nicht dargestellt) ist in dem vierten Beispiel in beispielsweise einer Ausnehmung entlang der Peripherie der Lagervorrichtung 70 aufgenommen, wobei insbesondere der verbaute auslesbare Datenspeicher 12 am Außenring eingelassen sein kann und gleichzeitig ausreichend Raum für eine spulenförmig ausgebildete und ebenfalls in den Außenring eingelassene Antenne 14 verbleibt. Zur fluid- und gasdichten Ausführung des Außenringraums kann dieser vorzugsweise mit beispielsweise einem Kunststoffmaterial oder dergleichen vergossen erzeugt sein. Besonders vorteilhaft bei diesem Beispiel kann sein, dass die RFID-Vorrichtung rotationsfrei bleibt, da der Außenring der Lagervorrichtung 70 fest in einem entsprechenden Gegenstück einer umgebenden Einbau- bzw. Gehäuseanordnung steht.

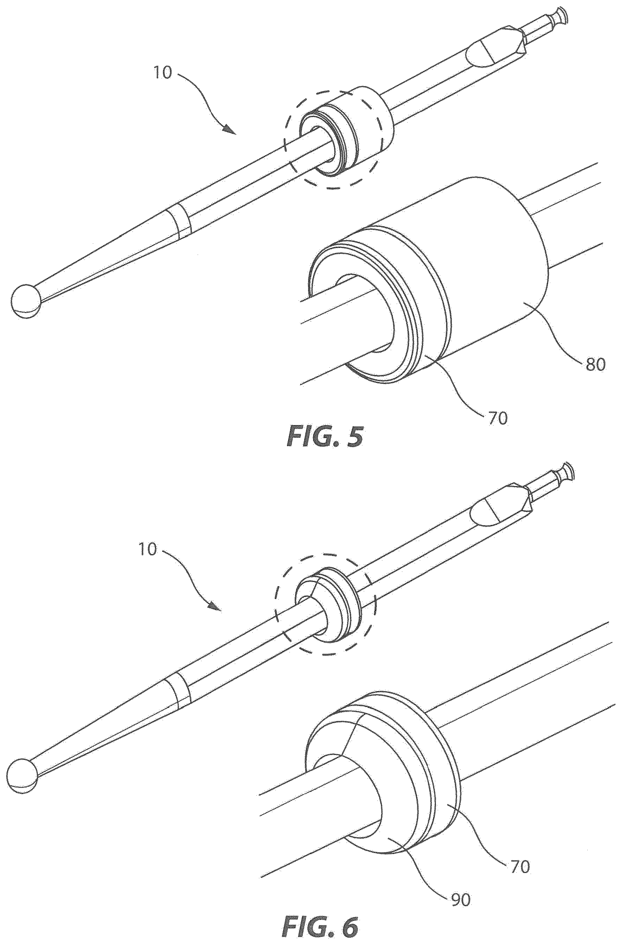

Fig. 5 stellt als ein fünftes Beispiel ebenfalls ein Werkzeug mit auslesbarem Datenspeicher dar, bei dem die RFID-Vorrichtung in einem Distanzhülsenelement 80 beispielsweise der entlang des Schaftabschnitts vorzugsweise fest verbaut angeordneten Lagervorrichtung 70 aufgenommen angeordnet ist, wobei die Distanzhülse als Träger für den RFID/NFC-Chip genutzt wird. Auch in diesem Beispiel ist genügend Bauraum bereitgestellt, um eine Antenne mit zu verbauen, so dass auch in diesem Beispiel die RFID-Vorrichtung mit Antenne und Transponderelektronik (nicht dargestellt) in beispielsweise einem Raum entlang der Peripherie des Distanzhülsenelements 80 aufgenommen sein kann, wobei insbesondere der verbaute auslesbare Datenspeicher (nicht dargestellt) in einem Ringraumabschnitt eingelassen sein kann und gleichzeitig ausreichend Raum für die spulenförmig ausgebildete und ebenfalls in den Ringraumabschnitt eingelassene Antenne (nicht dargestellt) verbleibt. Zur fluid- und gasdichten Ausführung des Ringraumabschnitts kann dieser vorzugsweise mit beispielsweise einem Harz- oder Kunststoffmaterial oder dergleichen vergossen erzeugt sein. Besonders vorteilhaft bei diesem Beispiel kann sein, dass die RFID-Vorrichtung rotationsfrei bleibt, wenn das Distanzhülsenelement 80 fest in einem entsprechenden Gegenstück einer umgebenden Einbau- bzw. Gehäuseanordnung steht.

Fig. 6 stellt als ein sechstes Beispiel ebenfalls ein Werkzeug mit auslesbarem Datenspeicher dar, bei dem die RFID-Vorrichtung in einem Spritzschutzelement 90 beispielsweise der entlang des Schaftabschnitts vorzugsweise fest verbaut angeordneten Lagervorrichtung 70 aufgenommen angeordnet ist, wobei das Spritzschutzelement 90 als Träger für den RFID/NFC-Chip genutzt wird. Auch in diesem Beispiel ist genügend Bauraum bereitgestellt, um eine Antenne mit zu verbauen, so dass auch in diesem Beispiel die RFID-Vorrichtung mit Antenne (nicht dargestellt) und Transponderelektronik (nicht dargestellt) in beispielsweise einem Raum entlang der Peripherie des Spritzschutzelements 90 aufgenommen sein kann, wobei insbesondere der verbaute auslesbare Datenspeicher (nicht dargestellt) in einem Ringraumabschnitt eingelassen sein kann und gleichzeitig ausreichend Raum für die spulenförmig ausgebildete und ebenfalls in den Ringraumabschnitt eingelassene Antenne (nicht dargestellt) verbleibt. Zur fluid- und gasdichten Ausführung des Ringraumabschnitts kann dieser vorzugsweise mit beispielsweise einem Harz- oder Kunststoffmaterial oder dergleichen vergossen erzeugt sein. Besonders vorteilhaft bei diesem Beispiel kann sein, dass die RFID-Vorrichtung rotationsfrei bleibt, wenn das Spritzschutzelement 90 fest in einem entsprechenden Gegenstück einer umgebenden Einbau- bzw. Gehäuseanordnung steht und/oder verdrehfest mit beispielsweise einer zu schützenden Lagervorrichtung 70 oder dergleichen gekoppelt ist.

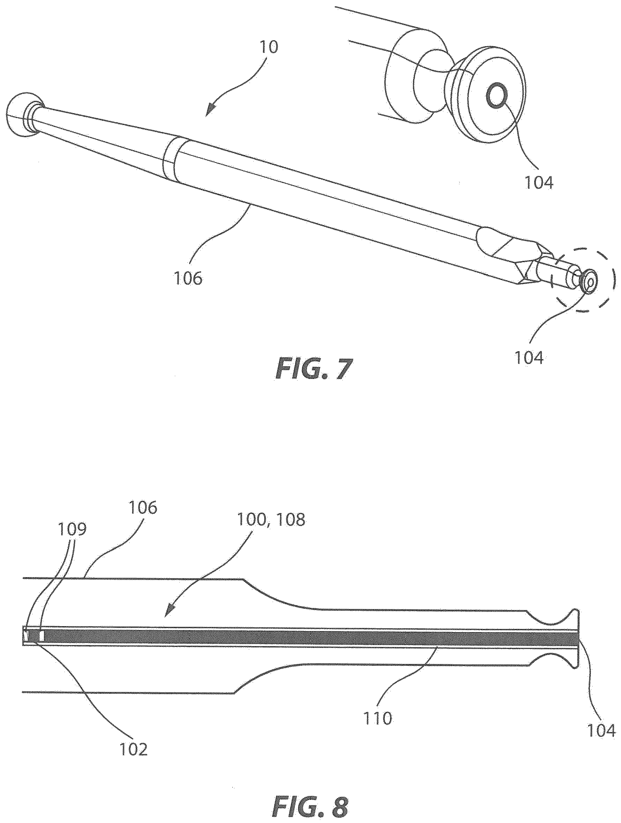

Fig. 7 zeigt in einer Ausführungsform als ein Ausführungsbeispiel des medizinischen Werkzeugs mit einer im Schaftbereich desselben angeordneten Arbeitsendenbestimmungsvorrichtung eine in dem Werkzeug 10 integrierte Einheit 100, die die Arbeitsendenbestimmungsvorrichtung bildet, mit einem das Arbeitsende des Werkzeugs bestimmenden Widerstand, der beispielsweise ein SMD-Widerstand oder dergleichen sein kann, und/oder einer Daten mit Bezug zu dem Arbeitsende speichernden Speichervorrichtung, die beispielsweise ein SMD-Speicherchip oder dergleichen sein kann, wobei die in dem Werkzeug 10 integrierte Einheit 100 über eine von der Einheit 100 nach außen geführte elektrische Kontaktierung 104 in einem Handstück 106 des Werkzeugs 10 mit einem externen Gerät (nicht dargestellt), beispielsweise einem Steuergerät oder einer Signal- und/oder Datenverarbeitungsvorrichtung, verbindbar ist.

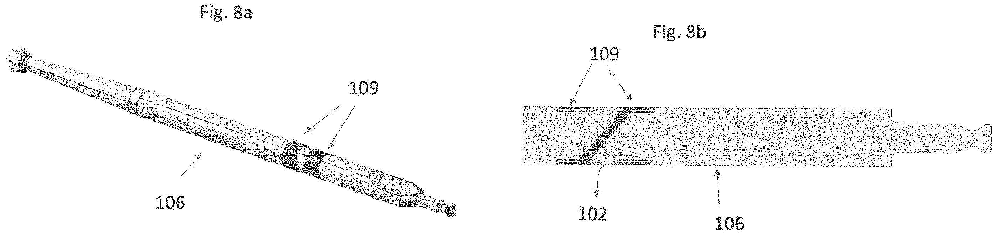

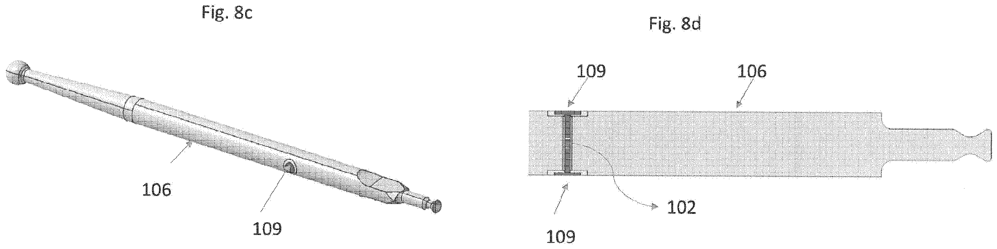

Fig. 8 stellt dazu in einer Schnittansicht durch den proximalen Endabschnitt 106 des Werkzeugs näher die Anordnung der integrierten Einheit 100 in dem Werkzeug 10 dar. Gemäß Fig. 8 erstreckt sich die in das Werkzeug 10 integrierte Einheit 100 ausgehend von einem griff- oder schaftseitigen proximalen Ende des Werkzeugs 10, an dem außenseitig des Werkzeugs die elektrische Kontaktierung 104 angeordnet ist, mit einer kanalförmigen Struktur 108 in Richtung hin zu dem distalen Arbeitsende in das Werkzeug hinein. In der kanalförmigen Struktur ist an einem ersten, dem distalen Arbeitsende (z.B. Fräskopf) zugewandten Ende der das Arbeitsende (z.B. Fräskopf) des Werkzeugs bestimmende Widerstand und/oder die Daten mit Bezug zu dem Arbeitsende (z.B. Fräskopf) speichernde Speichervorrichtung 102 angeordnet, und ist an einem zweiten, dem distalen Arbeitsende abgewandten Ende die elektrische Kontaktierung 104 angeordnet. Zumindest ein Leiterabschnitt kontaktiert den Widerstand und/oder die Speichervorrichtung 102 elektrisch. In der kanalförmigen Struktur verbinden Kontakte 109 den Widerstand und/oder die Speichervorrichtung 102 mit einem zu der elektrischen Kontaktierung 104 führenden Leiter 110 und einer potenzialseitigen Masse, und die kanalförmige Struktur verläuft innerhalb eines Isolators bzw. isolierenden Materials umhüllt, der bzw. das deren innenliegende Komponenten dem Werkzeug 10 elektrisch isoliert.

An dieser Stelle sei darauf hingewiesen, dass die Kontakte 109 gemäß der Fig. 8 rein funktional als Kontaktpunkte oder Streifen dargestellt sind. Die Fig. 8a und 8b zeigen hingegen eine mögliche konstruktive Ausgestaltung dieser Kontakte 109 vorzugsweise als Kontaktringe, die demzufolge in jeder beliebigen Rotationslage des Werkzeugs bezüglich einer Werkzeugaufnahme auf Seiten eines Griffs oder einer Antriebseinheit mit dort vorhandenen Kontaktstiften (nicht gezeigt) in Kontaktanlage gehalten werden können. Dabei kann der Widerstand 102 im Schaftinnern angeordnet sein und über eine Leitung eine elektrische Verbindung zu den Ringkontakten haben.

Eine alternative Konstruktion der Kontaktpunkte ist in den Fig. 8c und 8d dargestellt, wonach die Kontaktpunkte 109 als zwei an diametral voneinander abgewandten Schaftumfangspositionen (also gegenüberliegend) angeordnete Kontaktpads oder Kontaktflächen ausgebildet sind. Diese sind über eine quer verlaufende Leitung elektrisch miteinander verbunden, in die ein Widerstand zwischengeschaltet ist. Vorteilhaft kann die beschreibbare und/oder auslesbare Datenspeichervorrichtung zumindest einen zweiten Speicherbereich aufweisen, der dazu konfiguriert ist, andere Daten als die Daten mit Bezug zu dem Arbeitsende des Werkzeugs zu speichern.

Wie vorstehend beschrieben wurde, weist ein Werkzeug 10 eine in einem Schaftbereich desselben angeordnete Arbeitsendenbestimmungsvorrichtung zur Bestimmung und/oder Erkennung eines Arbeitsendes des Werkzeugs 10 auf, wobei die Arbeitsendenbestimmungsvorrichtung eine beschreibbare und/oder auslesbare Datenspeichervorrichtung beinhaltet, die dazu konfiguriert ist, in zumindest einem ersten Speicherbereich zumindest Daten mit Bezug zu dem Arbeitsende des Werkzeugs zu speichern, und wobei die Arbeitsendenbestimmungsvorrichtung dazu angeordnet ist, die Daten mit Bezug zu dem Arbeitsende des Werkzeugs zur Erkennung des Arbeitsendes des Werkzeugs extern erfassbar bereitzustellen. Die Arbeitsendenbestimmungsvorrichtung kann eine RFID-Vorrichtung sein, die zumindest die Datenspeichervorrichtung und eine Antennenvorrichtung umfasst. Alternativ kann die eine in das Werkzeug integrierte Einheit sein, die über eine elektrische Kontaktierung in einem Handstückbereich des Werkzeugs mit einem externen Steuergerät verbindbar ist, wobei die die in das Werkzeug integrierte Einheit einen das Arbeitsende des Werkzeugs bestimmenden Widerstand und/oder eine Daten mit Bezug zu dem Arbeitsende speichernde Speichervorrichtung umfasst. Somit können Arbeitsenden erkannt werden und je nach Ausführungsvariante auch Daten, die zuvor auf den auslesbaren Datenspeicher geschrieben wurden, ausgelesen und weiterverwendet werden.

Es versteht sich, dass die Erfindung nicht auf das vorstehend beschriebene Ausführungsbeispiel beschränkt ist, sondern Änderungen und Modifikationen im Rahmen des anspruchsgemäß definierten Schutzumfangs ebenfalls von der Erfindung umfasst sind. Beispielsweise sind als Anwendungsfälle pay per use-Anwendungen und Konsignationslageransätze denkbar, wofür sich dem Fachmann solche Änderungen und Modifikationen ohne Weiteres ergeben.

## Patentansprüche

1. Werkzeug mit einer in einem Schaftbereich desselben angeordneten Arbeitsendenbestimmungsvorrichtung zur Bestimmung eines Arbeitsendes des Werkzeugs (10), wobei die Arbeitsendenbestimmungsvorrichtung eine beschreibbare und/oder auslesbare Datenspeichervorrichtung beinhaltet, die dazu konfiguriert ist, in zumindest einem ersten Speicherbereich zumindest Daten mit Bezug zu dem Arbeitsende des Werkzeugs (10) zu speichern, und wobei die Arbeitsendenbestimmungsvorrichtung dazu angeordnet ist, die Daten mit Bezug zu dem Arbeitsende des Werkzeugs (10) zur Erkennung des Arbeitsendes des Werkzeugs (10) extern erfassbar bereitzustellen,
**dadurch gekennzeichnet, dass**
die Arbeitsendenbestimmungsvorrichtung eine in das Werkzeug integrierte Einheit (100) ist, die über eine elektrische Kontaktierung (104) in einem Handstückbereich des Werkzeugs (10) mit einem externen Steuergerät verbindbar ist.

2. Werkzeug nach Anspruch 1, bei dem die in das Werkzeug (10) integrierte Einheit (100) einen das Arbeitsende des Werkzeugs (10) bestimmenden Widerstand und/oder eine Daten mit Bezug zu dem Arbeitsende speichernde Speichervorrichtung (102) umfasst.

3. Werkzeug nach Anspruch 2, bei dem sich die in das Werkzeug (10) integrierte Einheit (100) ausgehend von einem griff- oder schaftseitigen Ende des Werkzeugs mit kanalförmiger Struktur (108) in Richtung hin zu dem Arbeitsende in das Werkzeug (10) erstreckt, wobei in der kanalförmigen Struktur (108) an einem ersten, dem Arbeitsende zugewandten Ende der das Arbeitsende des Werkzeugs (10) bestimmende Widerstand und/oder die Daten mit Bezug zu dem Arbeitsende speichernde Speichervorrichtung (102) angeordnet ist und an einem zweiten, dem Arbeitsende abgewandten Ende die elektrische Kontaktierung (104) angeordnet ist, wobei zumindest ein Leiterabschnitt (110) den Widerstand und/oder die Speichervorrichtung (102) elektrisch kontaktiert und wobei die kanalförmige Struktur (108) von einem Isolator (112) umhüllt und durch diesen gegenüber dem Werkzeug (10) elektrisch isoliert ist.

4. Werkzeug nach einem der vorangehenden Ansprüche, bei dem die beschreibbare und/oder auslesbare Datenspeichervorrichtung zumindest einen zweiten Speicherbereich beinhaltet, der dazu konfiguriert ist, vorbestimmte andere Daten als die in dem zumindest einen ersten Speicherbereich gespeicherten Daten, die die Daten mit Bezug zu dem Arbeitsende des Werkzeugs (10) sind, zu speichern.

## Claims

1. A tool having a working end determining device arranged in a shaft region thereof for determining a working end of the tool (10), wherein the working end determining device includes a writable and/or readable data storage device configured to store at least data related to the working end of the tool (10) in at least a first memory area, and wherein the working end determining device is arranged to provide the data related to the working end of the tool (10) in an externally detectable manner for recognizing the working end of the tool (10),
**characterized in that** the working end determination device is a unit (100) integrated into the tool and connectable to an external control device via an electrical contact (104) in a handpiece region of the tool (10).

2. The tool according to claim 1, wherein the unit (100) integrated in the tool (10) comprises a resistor determining the working end of the tool (10) and/or a storage device (102) storing data related to the working end.

3. The tool according to claim 2, wherein the unit (100) integrated in the tool (10) extends into the tool (10) starting from an end of the tool on the handle side or shaft side with a channel-shaped structure (108) in the direction towards the working end, wherein the resistor determining the working end of the tool (10) and/or the storage device (102) storing data related to the working end is arranged in the channel-shaped structure (108) at a first end facing the working end, and wherein the electrical contact (104) is arranged at a second end facing away from the working end, wherein at least one conductive section (110) makes electrical contact with the resistor and/or the storage device (102), and wherein the channel-shaped structure (108) is enclosed by an insulator (112) and is electrically insulated by the latter with respect to the tool (10).

4. The tool according to one of the preceding claims, wherein the writable and/or readable data storage device includes at least one second memory area configured to store predetermined data other than the data stored in the at least one first memory area, which is the data related to the working end of the tool (10).

## Revendications

1. Outil avec un dispositif de détermination d'extrémité de travail disposé dans une zone de tige de celui-ci pour déterminer une extrémité de travail de l'outil (10), dans lequel le dispositif de détermination d'extrémité de travail contient un dispositif de stockage de données inscriptible et/ou lisible qui est configuré pour stocker au moins des données relatives à l'extrémité de travail de l'outil (10) dans au moins une première zone de stockage, et dans lequel le
dispositif de détermination d'extrémité de travail est disposé pour fournir les données relatives à l'extrémité de travail de l'outil (10) de manière détectable de l'extérieur pour reconnaître l'extrémité de travail de l'outil (10),
**caractérisé en ce que** le dispositif de détermination d'extrémité de travail est une unité (100) intégrée dans l'outil qui peut être connectée à un appareil de commande extérieur par l'intermédiaire d'une mise en contact électrique (104) dans une zone de pièce à main de l'outil (10).

2. Outil selon la revendication 1, dans lequel l'unité (100) intégrée dans l'outil (10) comprend une résistance déterminant l'extrémité de travail de l'outil (10) et/ou un dispositif de stockage (102) stockant des données relatives à l'extrémité de travail.

3. Outil selon la revendication 2, dans lequel l'unité (100) intégrée dans l'outil (10) s'étend dans l'outil (10) en partant d'une extrémité de l'outil côté poignée ou tige avec une structure en forme de canal (108) en direction de l'extrémité de travail, dans lequel la résistance déterminant l'extrémité de travail de l'outil (10) et/ou le dispositif de stockage (102) stockant les données relatives à l'extrémité de travail sont disposés dans la structure en forme de canal (108) au niveau d'une première extrémité tournée vers l'extrémité de travail, et la mise en contact électrique (104) est disposée au niveau d'une seconde extrémité opposée à l'extrémité de travail, dans lequel au moins une section conductrice (110) est en contact électrique avec la résistance et/ou le dispositif de stockage (102), et dans lequel la structure en forme de canal (108) est entourée d'un isolateur (112) et est isolée électriquement de l'outil (10) par celui-ci.

4. Outil selon l'une quelconque des revendications précédentes, dans lequel le dispositif de stockage de données inscriptible et/ou lisible contient au moins une seconde zone de stockage qui est configurée pour stocker d'autres données prédéterminées que les données stockées dans la au moins une première zone de stockage qui sont les données relatives à l'extrémité de travail de l'outil (10).
